Europäisches Patentamt

European Patent Office

Office européen des brevets

(19)

(11) Veröffentlichungsnummer: **0 311 960 B1**

## (12) EUROPÄISCHE PATENTSCHRIFT

(45) Veröffentlichungstag der Patentschrift: **26.01.94**

(51) Int. Cl.5: **C07D 201/16**

(21) Anmeldenummer: **88116802.5**

(22) Anmeldetag: **11.10.88**

(54) **Verfahren zur Gewinnung von Caprolactam aus Leichtsiedern oder Schwersiedern der Caprolactamdestillation oder Gemischen derselben.**

(30) Priorität: **16.10.87 DE 3735054**

(43) Veröffentlichungstag der Anmeldung:
**19.04.89 Patentblatt 89/16**

(45) Bekanntmachung des Hinweises auf die
Patenterteilung:
**26.01.94 Patentblatt 94/04**

(84) Benannte Vertragsstaaten:
**BE CH DE ES FR GB IT LI NL**

(56) Entgegenhaltungen:
**EP-A- 0 022 161**
**US-A- 2 813 858**
**US-A- 3 379 028**

(73) Patentinhaber: **BASF Aktiengesellschaft**
**Carl-Bosch-Strasse 38**
**D-67063 Ludwigshafen(DE)**

(72) Erfinder: **Neubauer, Gerald, Dr.**
**Mozartstrasse 24**
**D-6940 Weinheim(DE)**
Erfinder: **de Decker, Emile**
**Schijndallei 13**
**B-2120 Schoten(BE)**
Erfinder: **Fuchs, Hugo, Dr.**
**Egellstrasse 28**
**D-6700 Ludwigshafen(DE)**
Erfinder: **Holzknecht, Bernhard, Dr.**
**Pfalzgrafenstrasse 11**
**D-6701 Ellerstadt(DE)**
Erfinder: **Ritz, Josef, Dr.**
**Osloer Weg 8**
**D-6700 Ludwigshafen(DE)**

**Beschreibung**

Caprolactam wird bei dessen Herstellung zum Zwecke der Reinigung durch Destillation von leichtsiedenen und schwersiedenen Produkten getrennt. Sowohl die leichtsiedenden als auch die schwersiedenen Produkte enthalten noch erhebliche Mengen an Caprolactam. Es ist angezeigt, dieses aus den Nebenströmen der Destillation zu gewinnen und zu verwerten.

Nach einem in der EP-Anmeldung 22 161 beschrieben Verfahren wird aus dem alkalischen Destillationsrückstand in einer ersten Stufe bei einer Sumpftemperatur von 130 bis 160°C unter vermindertem Druck zunächst Caprolactam abdestilliert und aus dem nunmehr verbleibenden Rückstand in einer zweiten Stufe bei einer Sumpftemperatur von 140 bis 180°C unter vermindertem Druck weiteres Caprolactam abdestilliert, das in einer dritten Stufe mit Säure behandelt wird und wiederum in die Reinigungsoperation für das Caprolactam aus der Beckmannschen Umlage zurückgeführt wird. Ein solches Verfahren ist technisch aufwendig und es gelingt nicht, im wünschenswerten Maß schwer abtrennbare Verbindungen zu eliminieren.

Die an und für sich naheliegende Abtrennung von Nebenprodukten aus den leichtsiedenen Anteilen durch Destillation ist wenig wirkungsvoll, da ein hoher Destillationsaufwand erforderlich ist und zudem die Siedepunkte der Nebenprodukte sehr nahe bei dem des Caprolactams liegen.

Aus der US-PS 2 813 858 ist auch schon bekannt. Caprolactam durch fraktionierte Kristallisation zu reinigen. Es wird jedoch kein Hinweis gegeben, wie bei der Reinigung von Leicht- oder Schwersiedern der Caprolactamdestillation oder deren Gemischen zu verfahren ist.

Es war deshalb die technische Aufgabe gestellt, Leicht- oder Schwersieder aus der Caprolactamdestillation oder Gemische derselben aufzuarbeiten und das darin enthaltene Caprolactam zu gewinnen und hierbei schwer abtrennbare Verbindungen weitgehend abzureichern um das so erhaltene Caprolactam wiederum ohne Nachteile verwenden zu können.

Diese technische Aufgabe wird gelöst in einem Verfahren zur Gewinnung von Caprolactam aus Leicht- oder Schwersiedern der Caprolactamdestillation oder Gemischen derselben welches folgende Schritte umfaßt:

a) Kristallisieren von Leicht- oder Schwersiedern der Caprolactamdestillation oder Gemische derselben unter Bildung eines gereinigten Caprolactamkristallisats und Mutterlauge

b) Abtrennen des gereinigten Caprolactamkristallisats unter Verbleib von Mutterlauge

c) Rückführung von 5 bis 90 Gew.% der Mutterlauge aus Stufe b) in die Stufe a) und Überführen des verbleibenden Teils der Mutterlauge in die nachfolgende Stufe d)

d) Kristallisieren des Teils der Mutterlauge aus der Stufe c) unter Bildung eines Caprolactamkristallisats und Mutterlauge

e) Abtrennen des Caprolactamkristallisats aus der Stufe d) und Rückführen desselben in die Stufe a) unter Verbleib von Mutterlauge

f) Rückführung von 20 bis 99 Gew.% der Mutterlauge aus der Stufe e) in die Stufe d) und Ausschleusen des verbleibenden Teils der die Verunreinigungen enthaltenden Mutterlauge aus der Stufe e).

Das neue Verfahren hat den Vorteil, daß es auf einfache Weise gelingt, Leichtsieder oder Schwersieder aus der Caprolactamdestillation einzeln oder gemeinsam unter Gewinnung von Caprolactam aufzuarbeiten. Die gemeinsame Aufarbeitung hat den Vorteil, daß keine getrennte Reinigungsoperationen notwendig sind. Weiter hat das neue Verfahren den Vorteil, daß auch schwer abtrennbare Verunreinigungen weitgehend abgereichert werden. Zudem hat das neue Verfahren den Vorteil, daß der größte Teil des in den Leicht- und Schwersiedern enthaltenen Caprolactams auf einfache Weise in wiederverwendbarer Form zurückgewonnen wird.

Erfindungsgemäß geht man von Leichtsiedern oder Schwersiedern oder deren Gemischen aus, die bei der Reinigung von Caprolactam anfallen. Solche Nebenströme der Destillation erhält man beispielsweise in einer mehrstufigen Destillation, wobei man die Leichtsieder über Kopf abtrennt und nach Abtrennen von reinem Caprolactam bei Sumpftemperaturen von 120 bis 150°C unter vermindertem Druck Schwersieder anfallen. Geeignete Leichtsieder und Schwersieder erhält man z.B. nach dem in der EP 22 161 beschriebenen Verfahren. Vorteilhaft werden die Leichtsieder und Schwersieder gemeinsam als Gemisch aufgearbeitet. Vorteilhaft enthält das Gemisch je Gewichtsteil Leichtsieder 0,1 bis 10 Gew.-Teile Schwersieder. Das so erhaltene Ausgangsgemisch enthält z.B. von 90 bis 99,9 Gew.% Caprolactam sowie von 0,1 bis 10 Gew.% Verunreinigungen. Unter den Verunreinigungen seien u.a. genannt: unterschiedliche Mengen an N-Phenylacetamid, N-Methylcapronamid, 6-Methylvalerolactam, 7-Methyllactam, 3-Methyllactam und Octahydrophenazin.

Caprolactam wird unter Abreicherung der Verunreinigungen aus den Leichtsiedern oder Schwersiedern der Caprolactamdestillation oder deren Gemische durch Kristallisation gewonnen. Hierfür eignen sich die in

EP 0 311 960 B1

der Technik üblichen Kristallisationsverfahren wie Schichtkristallisation, bei der man Caprolactam auf einer gekühlten Fläche als Kristallschicht ablagert und von der zurückbleibenden Mutterlauge trennt. Geeignet sind auch Kristallisationsverfahren, bei denen man unter Bewegung des zu kristallisierenden Mediums eine Kristallmaische, bestehend aus kristallisiertem Caprolactam und Mutterlauge, erzeugt und dann das auskristallisierte Caprolactam nach üblichen Methoden, z.B. durch Zentrifugieren, abtrennt. Besonders bewährt hat sich letzteres Verfahren als Vakuumkristallisation, wie es beispielsweise in der US-PS 2 813 858 beschrieben wird.

Erfindungsgemäß werden Leichtsieder oder Schwersieder oder deren Gemische in der Stufe a) unter Bildung eines gereinigten Caprolactamkristallisats und Mutterlauge kristallisiert. Vorteilhaft kristallisiert man bis zu einem Kristallisationsgrad von 20 bis 60 %, insbesondere 25 bis 40 %. Unter Kristallisationsgrad sei der prozentuale Gewichtsanteil an kristallisiertem Caprolactam, bezogen auf das eingesetzte Gemisch, verstanden. Der Rest ist die flüssige Mutterlauge. Es hat sich auch als vorteilhaft erwiesen, wenn man während der Kristallisation in der Stufe a) einen Wassergehalt von 5 bis 20 Gew.%, insbesondere 10 bis 15 Gew.%, bezogen auf das eingesetzte Gemisch, aufrecht erhält. Besonders vorteilhaft hält man bei einer Vakuumkristallisation verminderten Druck z.B. von 5 bis 150 mbar und eine Temperatur von 15 bis 60°C ein.

Das so erhaltene gereinigte Caprolactamkristallisat und die Mutterlauge werden in der Stufe b) getrennt. Falls eine Schichtkristallisation angewandt wird, läßt man die Mutterlauge ab und schmilzt das erhaltene Kristallisat getrennt auf. Sofern man z.B. Kristalle unter Durchmischung als Maische aus Caprolactamkristallisat und Mutterlauge erzeugt hat, wird das Caprolactamkristallisat vorzugsweise durch Zentrifugieren in Caprolactamkristallisat und Mutterlauge getrennt. Das so erhaltene gereinigte Caprolactamkristallisat wird zweckmäßig wiederum der Caprolactamherstellung zugeführt, z.B. in der Stufe der Extraktion des Rohlactams.

In Stufe c) werden 5 bis 90 Gew.% der in Stufe b) erhaltenen Mutterlauge, z.B. 10 bis 80 Gew.%, nach Stufe a) zur Kristallisation gemeinsam mit neuem Ausgangsgemisch zugeführt. Der verbleibende Teil der Mutterlauge aus Stufe b), d.h. der restliche Teil, z.B. 10 bis 95 Gew.%, wird der nachfolgenden Stufe d) zugeführt.

In der folgenden Stufe d) wird der Teil, der Mutterlauge aus c) unter Bildung eines Caprolactamkristallisats und Mutterlauge kristallisiert. Hierbei hält man vorteilhaft einen Kristallisationsgrad von 20 bis 60 % ein. Vorteilhaft wird die Kristallisation unter vermindertem Druck, z.B. 5 bis 150 mbar und einer Temperatur von 15 bis 60°C durchgeführt. Darüber hinaus wird während der Kristallisation zweckmäßig Wasser abdestilliert, z.B. 10 bis 50 Gew.% des in der eingesetzten Mutterlauge enthaltenen Wassers.

In der Stufe e) wird das Kristallisat und die Mutterlauge aus d) analog der Stufe b) in kristallisiertes Caprolactam und Mutterlauge getrennt. Das so erhaltene kristalline Caprolactam wird wiederum in die Stufe a) zurückgeführt und erneut mit frischem Ausgangsgemisch und Mutterlauge aus der Stufe c) kristallisiert.

In der Stufe f) werden 20 bis 99 Gew.% der die Verunreinigungen enthaltenden Mutterlauge aus der Stufe e), vorzugsweise 30 bis 98,5 Gew.%, abgetrennt und in die Stufe d) erneut zur Kristallisation zurückgeführt, während der verbleibende Teil der Mutterlauge aus der Stufe e), zur Beseitigung der Verunreinigungen ausgeschleust wird.

Besonders bewährt hat sich die Vakuumkristallisation, bei der
in der Stufe a) Leicht- oder Schwersiedern oder deren Gemische unter Aufrechterhaltung eines Wassergehalts von 5 bis 20 Gew.% unter einem Druck von 5 bis 150 mbar bei einer Temperatur von 15 bis 60°C unter Bildung einer Maische aus 20 bis 60 Gew.% gereinigtem Caprolactamkristallisat und Mutterlauge kristallisiert wird
in der Stufe b) aus der in a) erhaltenen Maische das gereinigte kristallisierte Caprolactam, z.B. durch Zentrifugieren, abgetrennt und ausgeschleust wird unter Verbleib von Mutterlauge.

In der Stufe c) werden von der in Stufe b) anfallenden Mutterlauge 10 bis 80 Gew.% in die Stufe a) zurückgeführt und der verbleibende Teil der Mutterlauge in die nachfolgende Stufe d) geführt.

In der Stufe d) wird der verbleibende Teil der Mutterlauge aus der Stufe c) unter einem Druck von 5 bis 150 mbar bei einer Temperatur von 15 bis 60°C unter Abdestillieren von 10 bis 50 Gew.% des in der Mutterlauge enthaltenen Wassers unter Bildung einer Maische aus 25 bis 55 Gew.% kristallisiertem Caprolactam und Mutterlauge kristallisiert.

In der nachfolgenden Stufe e) wird aus der in Stufe d) anfallende Maische kristallisiertes Caprolactam abgetrennt und in die Stufe a) zurückgeführt unter Verbleib von Mutterlauge.

f) Von der in der Stufe e) anfallenden Mutterlauge werden 30 bis 98,5 Gew.% in die Kristallisationsstufe d) zurückgeführt und der restliche Teil der die Verunreinigungen enthaltenen Mutterlauge ausgeschleust.

Das Verfahren nach der Erfindung sei an folgendem Beispiel veranschaulicht.

Beispiel

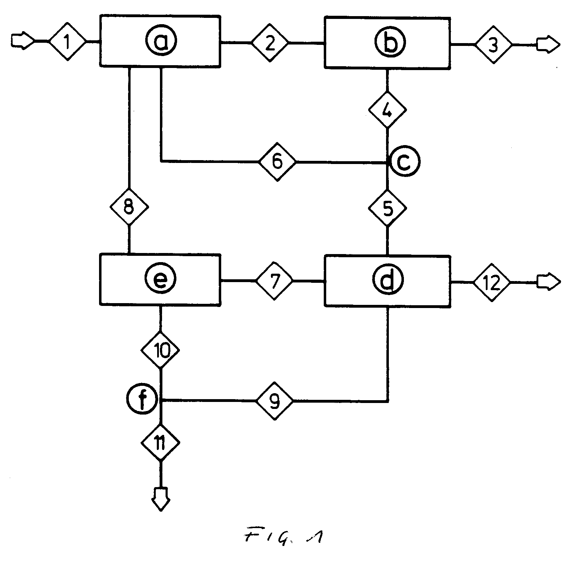

Eine Mischung aus Vorlauf (Leichtsieder) und Sumpfprodukt (Schwersieder) einer Lactamdestillationskolonne im Verhältnis 1:1,5 wird gemäß Figur 1 in einer Menge von 114,6 kg pro Stunde und folgender Zusammensetzung über I der 1. Kristallistiaonsstufe a) zugefügt:

100 kg Lactam

1,8 kg Verunreinigungen (Leicht- und Schwersieder)

12,8 kg Wasser.

Über die Leitung 6 wird der 1. Kristallisationsstufe eine Mischung aus

71,4 kg Lactam

5,8 kg Verunreinigungen

9,4 kg Wasser

aus der Stufe e) zugeführt. Kristallisat aus der 2. Kristallerstufe d) folgender Zusammensetzung wird über Leitung 8 in den Kristaller 1 geführt

99,5 kg Lactam

6,8 kg Verunreinigungen

1,3 kg Wasser.

In der 1. Kristallisationsstufe wird die Kristallisation bei 35°C und 20 mbar ausgeführt. Die Zulauftemperatur beträgt etwa 40°C. Die Temperatur wird durch Siedekühlung eingehalten. Der Kristaller wird mit 90 upm gerührt.

Die anfallende Maische (30 Gew.% Kristallisat) gelangt über 2 in eine Schubzentrifuge (b) mit 2000 upm. Das hierbei anfallende Kristallisat der Zusammensetzung

98 kg Lactam

0,4 kg Verunreinigungen

0,7 kg Wasser

wird ausgeschleust und gelangt in den Hauptstrom der Lactamreinigung der Caprolactamherstellung zurück.

Die beim Zentrifugieren (b) anfallende Mutterlauge wird in einer Menge von (59 Gew.% der Mutterlauge)

101,5 kg Lactam

8,2 kg Verunreinigungen

13,4 kg Wasser

über Leitung 5 der 2. Kristallisationsstufe (d) zugeführt.

Über Leitung 6 wird die bereits beschriebene Mutterlauge (41 Gew.%) in die 1. Kristallisationsstufe (a) gebracht.

Die in der Kristallisationsstufe d anfallende Maische wird in einer Zentrifuge (e) getrennt und der Kristallisatanteil über Leitung 8 wie beschrieben in die Stufe a) zugeführt.

Während die Mutterlauge in einer Menge von

102,7 kg Lactam

71,5 kg Verunreinigungen

13,5 kg Wasser

(98 Gew.%) über die Leitung 9 der 2. Kristallisationsstufe d) zugeführt wird, werden

2 kg Lactam

1,4 kg Verunreinigungen

0,3 kg Wasser

der Mutterlauge über Leitung 11 ausgeschleust und verworfen.

Zur Aufrechterhaltung der Wasserkonzentration werden in der Stufe d) unter den Bedingungen der Stufe 1, 11,8 kg Wasser (9,6 Gew.%) verdampft und ausgeschleust.

Zur Charakterisierung des Einsatzproduktes im Vergleich zum Kristallisat sind beim Lactam übliche Kennzahlen sowie die durch Destillation schwer abtrennbaren Verunreinigungen angegeben.

4

```
Einsatzprodukt                              Kristallisat

fl. Basen meg/kg                3,36              0,8

GC Verunreinigungen ppm         552              130

davon waren unter anderem

N-Pentylacetamid                160              40

N-Methylcapronamid               47              10

6-Methylvalerolactam             78              18

7-Methyllactam                   90              21

3-Methyllactam                   38               8

Oktahydrophenazin              1,7 ppm           0,3
```

Vergleichsbeispiel 1

Führt man die destillative Trennung von Leichtsiedern in einer Kolonne mit einer Füllkörperschicht von 10 m (Pallringe) bei einem Kopfdruck von 10 mbar und einem Rücklaufverhältnis von 40 durch, so ist bei Verunreinigungen, die ihren Siedepunkt in der Nähe des Lactams haben, jedoch nur eine unvollständige Abtrennung möglich.

| Zulauf zur Leichtsiederentfernung | | Destillationsrückstand nach der Leichtsiederentfernung |
|---|---|---|
| fl. Basen meg/kg | 2,3 | 2,3 |
| GC Verunreinigungen ppm davon waren u.a. | 1200 | 900 |
| N-Pentylacetamid | 300 | 220 |
| N-Methylcapronamid | 90 | 65 |
| 6-Methylvalerolactam | 190 | 160 |
| 7-Methyllactam | 220 | 190 |
| 3-Methyllactam | 90 | 85 |
| Oktahydrophenazin | 0,5 | 0,5 |

Während die Abreicherung der GC-Verunreinigungen bei der Destillation nur geringfügig war, nahmen die durch Destillation besonders schwer abtrennbaren Verunreinigungen bei der Kristallisation um den Faktor ≈4 ab.

Vergleichsbeispiel 2

Bei einer Destillation des Schwersieder enthaltendem Lactams durch Destillation blieb die Menge des besonders schwer abtrennbaren Oktahydrophenazins im Destillat praktisch unverändert und brachte folgendes Ergebnis:

| | Zulauf | Destillat |
|---|---|---|
| GC Verunreinigungen ppm | 220 | 200 |
| Oktahydrophenazin | 2,5 | 2,2 |

**Patentansprüche**

1. Verfahren zur Gewinnung von Caprolactam aus Leichtsiedern oder Schwersiedern der Caprolactamdestillation oder Gemischen derselben, welches folgende Schritte umfaßt:
   a) Kristallisieren von Leichtsiedern oder Schwersiedern oder deren Gemische unter Bildung eines gereinigten Caprolactamkristallisats und Mutterlauge

b) Abtrennen des gereinigten Caprolactamkristallisats unter Verbleib von Mutterlauge

c) Rückführung von 5 bis 90 Gew.% der Mutterlauge aus Stufe b) in die Stufe a) und Überführen des verbleibenden Teils der Mutterlauge in die nachfolgende Stufe d)

d) Kristallisieren des Teils der Mutterlauge aus der Stufe c) unter Bildung eines Caprolactamkristallisats und Mutterlauge

e) Abtrennen des Caprolactamkristallisats aus der Stufe d) und dessen Rückführung in die Stufe a) unter Verbleib von Mutterlauge

f) Rückführung 20 bis 99 Gew.% der Mutterlauge aus der Stufe e) in die Stufe d) und Ausschleusen des anderen Teils der die Verunreinigungen enthaltenden Mutterlauge aus der Stufe e).

2. Verfahren nach Anspruch 1, dadurch gekennzeichnet, daß man in den Stufen a) und d) einen Kristallisationsgrad von 20 bis 60 % einhält.

3. Verfahren nach den Ansprüchen 1 und 2, dadurch gekennzeichnet, daß man in der Stufe c) 10 bis 80 Gew.% der Mutterlauge in die Stufe a) zurückführt und den Rest der Stufe d) zuführt.

4. Verfahren nach den Ansprüchen 1 bis 3, dadurch gekennzeichnet, daß man in der Stufe f) 30 bis 98,5 Gew.% Mutterlauge in die Stufe d) zurückführt und den Rest ausschleust.

5. Verfahren nach den Ansprüchen 1 bis 4, dadurch gekennzeichnet, daß man in der Stufe a) einen Wassergehalt von 5 bis 20 Gew.% einhält.

6. Verfahren nach den Ansprüchen 1 bis 5, dadurch gekennzeichnet, daß man die Kristallisation in den Stufen a) und d) unter vermindertem Druck bei einer Temperatur von 15 bis 60°C durchführt.

7. Verfahren nach den Ansprüchen 1 bis 6, dadurch gekennzeichnet, daß man folgende Schritte durchführt:

a) Kristallisieren von Leichtsiedern oder Schwersiedern oder deren Gemische unter Aufrechterhaltung eines Wassergehaltes von 5 bis 20 Gew.% unter einem Druck von 5 bis 150 mbar bei einer Temperatur von 15 bis 60°C unter Bildung einer Maische aus 20 bis 60 Gew.% gereinigtem Caprolactamkristallisat und Mutterlauge

b) Abtrennen des gereinigten Caprolactamkristallisats unter Verbleib von Mutterlauge

c) Rückführen von 10 bis 80 Gew.% der Mutterlauge aus Stufe b) in die Stufe a) und Überführen des verbleibenden Teils der Mutterlauge in die nachfolgende Stufe d)

d) Kristallisieren des verbleibenden Teils der Mutterlauge aus der Stufe c) unter einem Druck von 5 bis 150 mbar bei einer Temperatur von 15 bis 60°C unter Abdestillieren von 10 bis 50 Gew.% des in der Mutterlauge enthaltenen Wassers unter Bildung einer Maische aus 25 bis 55 Gew.% kristallisiertem Caprolactam und der Mutterlauge

e) Abtrennen des kristallisierten Caprolactams aus der in Stufe d) erhaltenen Maische und Rückführung des Caprolactamkristallisats in die Stufe a) unter Verbleib von Mutterlauge

f) Rückführung von 30 bis 98,5 Gew.% der Mutterlauge aus der Stufe e) in die Stufe d) und Ausschleusen der restlichen, die Verunreinigungen enthaltenden, Mutterlauge.

8. Verfahren nach den Ansprüchen 1 bis 7, dadurch gekennzeichnet, daß man Gemische von Leichtsiedern und Schwersiedern der Caprolactamdestillation verwendet.

**Claims**

1. A process for recovering caprolactam from a caprolactam distillation low boiler or high boiler or a mixture thereof, comprising the following steps:

a) crystallizing a low or high boiler or a mixture thereof to form purified caprolactam crystals and a mother liquor,

b) separating off the purified caprolactam crystals to leave a mother liquor,

c) recycling from 5 to 90 % by weight of the mother liquor of stage b) into stage a) and transferring the remainder of the mother liquor into the subsequent stage d),

d) crystallizing the mother liquor portion from stage c) to form caprolactam crystals and a mother liquor,

e) separating off the caprolactam crystals of stage d) and recycling the same into stage a) to leave a mother liquor,
f) recycling from 20 to 99% by weight of the mother liquor of stage e) into stage d) and channeling out the remainder of the impurity-containing mother liquor of stage e).

2. A process as claimed in claim 1, wherein a degree of crystallization of from 20 to 60% is maintained in stages a) and d).

3. A process as claimed in claim 1 or 2, wherein in stage c) from 10 to 80% by weight of the mother liquor is recycled into stage a) and the remainder is passed into stage d).

4. A process as claimed in any of claims 1 to 3, wherein in stage f) from 30 to 98.5% by weight of the mother liquor is recycled into stage d) and the remainder is channeled out.

5. A process as claimed in any of claims 1 to 4, wherein in stage a) a water content of from 5 to 20% by weight is maintained.

6. A process as claimed in any of claims 1 to 5, wherein the crystallization in stages a) and d) is carried out under reduced pressure at from 15 to 60°C.

7. A process as claimed in any of claims 1 to 6, wherein the following steps are carried out:
   a) crystallizing a low boiler or a high boiler or a mixture thereof under from 5 to 150 mbar at from 15 to 60°C while maintaining a water content of from 5 to 20% by weight to form a slurry comprising from 20 to 60% by weight of purified caprolactam crystals and a mother liquor,
   b) separating off the purified caprolactam crystals to leave a mother liquor,
   c) recycling from 10 to 80% by weight of the mother liquor of stage b) into stage a) and transferring the remainder of the mother liquor into the subsequent stage d),
   d) crystallizing the remainder of the mother liquor from stage c) under from 5 to 150 mbar at from 15 to 60°C with distillative removal of from 10 to 50% by weight of the water contained in the mother liquor to form a slurry comprising from 25 to 55% by weight of crystalline caprolactam and the mother liquor,
   e) separating the crystalline caprolactam from the slurry obtained in stage d) and recycling the caprolactam crystals into stage a), leaving behind a mother liquor, and
   f) recycling from 30 to 98.5% by weight of the mother liquor of stage e) into stage d) and channeling out the remainder of the impurity-containing mother liquor.

8. A process as claimed in any of claims 1 to 7, wherein a mixture of caprolactam distillation low boilers and high boilers is used.

**Revendications**

1. Procédé de récupération de caprolactame à partir des fractions à haut ou bas point d'ébullition de la distillation du caprolactame, ou de mélanges de ces dernières, qui comprend les étapes suivantes :
   a) cristallisation des fractions à haut ou bas point d'ébullition ou de leurs mélanges, avec formation d'un caprolactame cristallisé purifié et d'une liqueur mère,
   b) séparation du caprolactame cristallisé purifié, en laissant la liqueur mère,
   c) recyclage de 5 à 90 % en poids de la liqueur mère de l'étape b) dans l'étape a) et transfert de la partie restante de la liqueur mère dans l'étape suivante d),
   d) cristallisation de la partie de la liqueur mère provenant de l'étape c), avec formation de caprolactame cristallisé et d'une liqueur mère,
   e) séparation du caprolactame cristallisé de l'étape d), et son recyclage dans l'étape a), en laissant une liqueur mère,
   f) recyclage de 20 à 99 % en poids de la liqueur mère de l'étape e) dans l'étape d), et soutirage de l'autre partie de la liqueur mère, contenant des impuretés, provenant de l'étape e).

2. Procédé selon la revendication 1, caractérisé en ce qu'on maintient dans les étapes a) et d) un degré de cristallisation de 20 à 60 %.

**3.** Procédé selon les revendications 1 et 2, caractérisé en ce que, dans l'étape c), on renvoie dans l'étape a) 10 à 80 % en poids de la liqueur mère, le reste étant envoyé à l'étape d).

**4.** Procédé selon les revendications 1 à 3, caractérisé en ce que, dans l'étape f), on recycle dans l'étape d) 30 à 98,5 % en poids de la liqueur mère, et on soutire le reste.

**5.** Procédé selon les revendications 1 à 4, caractérisé en ce qu'on maintient, dans l'étape a), une teneur en eau de 5 à 20 % en poids.

**6.** Procédé selon les revendications 1 à 5, caractérisé en ce qu'on met en oeuvre la cristallisation des étapes a) et d) sous pression réduite et à une température de 15 à 60°C.

**7.** Procédé selon les revendications 1 à 6, caractérisé en ce qu'on met en oeuvre les étapes suivantes:
a) cristallisation des fractions de haut ou bas point d'ébullition ou de leurs mélanges, tout en maintenant la teneur en eau à une valeur de 5 à 20 % en poids sous une pression de 5 à 150 mbar et à une température de 15 à 60°C, avec formation d'une pâte constituée de 20 à 60 % en poids de caprolactame cristallisé purifié et d'une liqueur mère,
b) séparation du caprolactame cristallisé purifié, en laissant la liqueur mère,
c) recyclage dans l'étape a), de 10 à 80 % en poids de la liqueur mère provenant de l'étape b) et transfert de la partie restante de la liqueur mère dans l'étape suivante d),
d) cristallisation de la partie restante de la liqueur mère provenant de l'étape c) sous une pression de 5 à 150 mbar et à une température de 15 à 60°C, en chassant par distillation 10 à 50 % en poids de l'eau contenue dans la liqueur mère, avec formation d'une pâte constituée de 25 à 55 % en poids de caprolactame cristallisé et de la liqueur mère,
e) séparation du caprolactame cristallisé de la pâte obtenue dans l'étape d), et renvoi du caprolactame cristallisé dans l'étape a) en laissant la liqueur mère,
f) recyclage de 30 à 98,5 % en poids de la liqueur mère provenant de l'étape e) dans l'étape d), et soutirage de la liqueur mère restante, qui contient les impuretés.

**8.** Procédé selon les revendications 1 à 7, caractérisé en ce qu'on utilise des mélanges de produits de haut ou bas point d'ébullition de la distillation du caprolactame.

FIG. 1